# EUROPEAN PATENT APPLICATION

(11) **EP 0 733 364 A1**
(43) Date of publication of application: **25.09.1996**
(21) Application number: 95900281.7
(22) Date of filing: 10.11.1994
(51) Int. Cl.: A61K 31/445

(54) **MEDICINAL COMPOSITION FOR CIRCULATORY ORGAN**

(30) Priority: 12.11.1993 JP 283090/93
(71) Applicant: NIPPON SHINYAKU COMPANY, LIMITED, Minami-ku Kyoto-shi Kyoto 601 (JP)
(72) Inventor: YOSHIKUNI, Yoshiaki, Kyoto 611 (JP); YAMAMOTO, Kiyoshi, Kyoto 619-02 (JP)
(74) Representative: Vogeser, Werner, Dipl.-Ing.
(86) International application number: JP9401898
(87) International publication number: WO9513068

(57) **Abstract**

A medicinal composition useful for treating thrombosis and reduced in side effects such as diarrhea, which contains a compound represented by general formula (II) as the active ingredient, wherein R⁶, R⁷, R⁸ and R⁹ represent the same C₁-C₈ acyl groups.

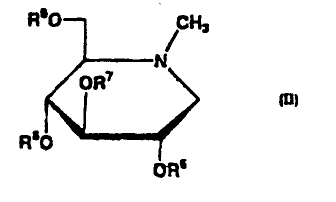

## Description

### TECHNICAL FIELD

The present invention relates to pharmaceutical compositions for the treatment of thrombus and other indications, each comprising a compound of the following general formula [I] or a physiologically acceptable salt thereof as an active ingredient. 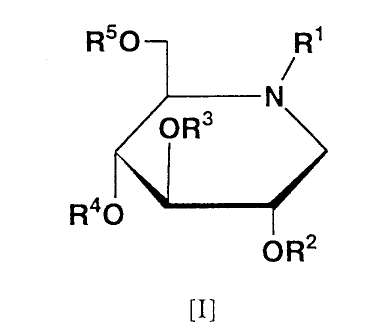 wherein R¹ represents hydrogen, alkyl, acyl, carboxyalkyl, alkyloxycarbonylalkyl, hydroxyalkyl, cycloalkylalkyl, arylalkyl, aryloxyalkyl, alkenyl, hydroxyalkenyl, arylalkenyl, aryloxyalkenyl, aryloxyarylalkenyl or alkylcarbamoylalkyl; R², R³, R⁴, and R⁵ may be the same or different and each represents hydrogen, alkyl, alkenyl or acyl (exclusive of the case in which all of R², R³, R⁴, and R⁵ represent hydrogen).

### BACKGROUND TECHNOLOGY

The living body is endowed with a mechanism for acting against excessive fibrinolysis. The factor which plays a cardinal role in this mechanism is alpha 2-plasmin inhibitor (hereinafter referred to briefly as α₂-PI).

α₂-PI rapidly inhibits plasmin activity in blood to prevent dissolution of a hemostatic plug and, as taken up in the plug, stabilize the thrombus. Moreover, α₂-PI is increased postoperatively as an acute-phase protein and, therefore, can be a cause of postoperative thrombosis. Therefore, if the blood α₂-PI activity could be decreased to change the balance of the fibrinolysis-coagulation system in favor of fibrinolysis, it would contribute to the prevention of postoperative thrombus formation and thrombosis and, at the same time, assist in the therapy of thrombus-associated illnesses such as myocardial and pulmonary infarcts.

As a class of compounds which decrease α₂-PI activity in blood to display thrombolytic efficacy, N-substituted moranoline compounds are known (Japanese Patent Application Kokai Heisei 1-250350). However, these N-substituted moranolines inhibit glucosidase activity in the gastrointestinal system as well and, therefore, have side effects such as diarrhea.

Meanwhile, O-acyl derivatives of N-substituted moranolines are known to have antiviral activity (Japanese Patent Application Kokai Heisei 2-172975). However, it has not been known to this day whether such O-acyl derivatives of N-substituted moranolines inhibit α₂-PI activity and, hence, have thrombolytic activity.

### DISCLOSURE OF INVENTION

The present invention has for its object to provide a pharmaceutical composition which is typically useful for the therapy of thrombosis and has a minimal risk of side effects such as diarrhea.

The inventors of the present invention having done much research found a series of compounds which help accomplish the above object and have perfected the present invention.

The series of compounds in the present invention (hereinafter referred to collectively as the compounds of the invention) are structurally characterized, as can be seen from the above general formula [I], in that N-substituted moranoline compounds have been typically O-alkylated, O-acylated or O-alkenylated.

The present invention is now described in detail.

Referring to the above general formula [I], R¹, R², R³, R⁴, and R⁵ represent the substituent groups defined generically as above.

Specifically, the alkyl mentioned for R¹ means a straight or branched-chain alkyl group of about 1 to 10 carbon atoms, thus typically including lower alkyl groups such as methyl, ethyl, propyl, isopropyl, butyl, isobutyl, sec-butyl, tert-butyl, etc., intermediate alkyl groups such as pentyl, isopentyl, hexyl, isohexyl, etc., and higher alkyl groups such as decyl. Among these groups, lower alkyl groups such as methyl, ethyl, propyl, isopropyl, butyl, isobutyl, etc. are preferred.

The acyl mentioned for R¹ is a straight or branched-chain acyl group of about 1 to 20 carbon atoms. Thus, formyl, acetyl, propionyl, isopropionyl, butyryl, isobutyryl, valeryl, isovaleryl, pivaloyl, hexanoyl, octanoyl, lauroyl, aroyl(benzoyl, toluoyl, α-naphthoyl, β-naphthoyl, 2-nicotinoyl, 3-nicotinoyl, 4-nicotinoyl, etc.), acryloyl, methacryloyl, oxalo, methoxalyl, ethoxalyl, cyclohexylcarbonyl, benzylcarbonyl, halo(fluoro, chloro, bromo or iodo)benzylcarbonyl, 4-phenylbenzylcarbonyl, phenoxycarbonyl, 2-phenylethylcarbonyl, cinnamoyl, methylmalonyl, ethylmalonyl, and propylmalonyl can be typically mentioned. Acyl species containing more carbon atoms also fall within the purview of the compounds of the invention.

The carboxyalkyl mentioned for R¹ includes but is not limited to carboxymethyl, 1-carboxyethyl, 2-carboxyethyl, 1-carboxypropyl, 2-carboxypropyl, and 3-carboxypropyl. Carboxyalkyl groups whose alkyl moieties contain more carbon atoms are also included in the purview of the compounds of the invention.

The alkyloxycarboxyalkyl mentioned for R¹ includes but is not limited to methoxycarbonylmethyl, 2-methoxycarbonylethyl, 2-methoxycarbonylpropyl, 3-methoxycarbonylpropyl, 2-methoxycarbonylbutyl, 3-methoxycarbonylbutyl, 4-methoxycarbonylbutyl, ethoxycarbonylmethyl, 2-ethoxycarbonylethyl, 2-ethoxycarbonylpropyl, 3-ethoxycarbonylpropyl, 2-ethoxycarbonylbutyl, 3-ethoxycarbonylbutyl, and 4-ethoxycarbonylbutyl. Species whose alkyl moieties contain more carbon atoms are also included in the purview of the compounds of the invention.

The hydroxyalkyl mentioned for R¹ includes but is not limited to hydroxymethyl, 1-hydroxyethyl, 2-hydroxyethyl, 1-hydroxypropyl, 2-hydroxypropyl, 3-hydroxypropyl, 1-hydroxybutyl, 2-hydroxybutyl, 3-hydroxybutyl, and 4-hydroxybutyl. Species whose alkyl moieties contain more carbon atoms are also included in the purview of the compounds of the invention.

The cycloalkylalkyl mentioned for R¹ is a group of about 3-10 carbon atoms, thus including cyclopropylmethyl, cyclobutylmethyl, cyclopentylmethyl, and cyclohexylmethyl, among others. Species containing more carbon atoms in the alkyl moiety are also included in the purview of the compounds of the invention.

The arylalkyl mentioned for R¹ is a group of about 7-20 carbon atoms, thus including but being not limited to benzyl, phenethyl, 1-phenylpropyl, 2-phenylpropyl, 3-phenylpropyl, 1-phenylbutyl, 2-phenylbutyl, 3-phenylbutyl, 4-phenylbutyl, 1-phenylpentyl, 2-phenylpentyl, 3-phenylpentyl, 4-phenylpentyl, 5-phenylpentyl, α-naphthylmethyl, β-naphthylmethyl, 1-(α-naphthyl)ethyl, 2-(α-naphthyl)ethyl, 1-(β-naphthyl)ethyl, 2-(β-naphthyl)ethyl, 1-(α-naphthyl)propyl, 2-(α-naphthyl)propyl, 3-(α-naphthyl)propyl, 1-(β-naphthyl)propyl, 2-(β-naphthyl)propyl, 3-(β-naphthyl)propyl, 1-(α-naphthyl)butyl, 2-(α-naphthyl)butyl, 3-(α-naphthyl)butyl, 4-(α-naphthyl)butyl, 1-(β-naphthyl)butyl, 2-(β-naphthyl)butyl, 3-(β-naphthyl)butyl, and 4-(β-naphthyl)butyl. Species containing more carbon atoms in the alkyl moiety are also included in the purview of the compounds of the invention.

The aryloxyalkyl mentioned for R¹ is a group of about 7-21 carbon atoms, thus including but being not limited to phenoxymethyl, 1-phenoxyethyl, 2-phenoxyethyl, 1-phenoxypropyl, 2-phenoxypropyl, 3-phenoxypropyl, 1-phenoxybutyl, 2-phenoxybutyl, 3-phenoxybutyl, 4-phenoxybutyl, α-naphthyloxymethyl, β-naphthyloxymethyl, 1-(α-naphthyloxy)ethyl, 2-(α-naphthyloxy)ethyl, 1-(β-naphthyloxy)ethyl, 2-(β-naphthyloxy)ethyl, 1-(α-naphthyloxy)propyl, 2-(α-naphthyloxy)propyl, 3-(α-naphthyloxy)propyl, 1-(β-naphthyloxy)propyl, 2-(β-naphthyloxy)propyl, 3-(β-naphthyloxy)propyl, 1-(α-naphthyloxy)butyl, 2-(α-naphthyloxy)butyl, 3-(α-naphthyloxy)butyl, 4-(α-naphthyloxy)butyl, 1-(β-naphthyloxy)butyl, 2-(β-naphthyloxy)butyl, 3-(β-naphthyloxy)butyl, and 4-(β-naphthyloxy)butyl. Species containing more carbon atoms are also included in the purview of the compounds of the invention.

The alkenyl mentioned for R¹ is a straight or branched-chain group of 2-20 carbon atoms, thus including but being not limited to vinyl, 1-propenyl, - 2-propenyl, isopropenyl, 1-butenyl, 2-butenyl, 3-butenyl, 2-methyl-1-propenyl, 2-methyl-2-propenyl, 1-methyl-1-propenyl, 1-methyl-2-propenyl, tert-butenyl, geranyl, and farnesyl. Species containing more carbon atoms are also included in the purview of the compounds of the invention.

The hydroxyalkenyl mentioned for R¹ includes 1-hydroxyvinyl, 2-hydroxyvinyl, 1-hydroxy-1-propenyl, 2-hydroxy-1-propenyl, 3-hydroxy-1-propenyl, 1-hydroxy-2-propenyl, 2-hydroxy-2-propenyl, 3-hydroxy-2-propenyl, 1-hydroxy-2-butenyl, 2-hydroxy-2-butenyl, 3-hydroxy-2-butenyl, 4-hydroxy-2-butenyl, 1-hydroxy-3-butenyl, 2-hydroxy-3-butenyl, 3-hydroxy-3-butenyl, and 4-hydroxy-3-butenyl, among others. Species containing more carbon atoms are also included in the purview of the compounds of the invention.

The arylalkenyl mentioned for R¹ is a group of about 8-20 carbon atoms. Thus, 1-phenylvinyl, 2-phenylvinyl, 1-phenyl-1-propenyl, 1-phenyl-2-propenyl, 2-phenyl-1-propenyl, 2-phenyl-2-propenyl, 3-phenyl-1-propenyl, 3-phenyl-2-propenyl, 3-phenyl-1-butenyl, 3-phenyl-2-butenyl, 3-phenyl-3-butenyl, 4-phenyl-1-butenyl, 4-phenyl-2-butenyl, 4-phenyl-3-butenyl, 2-(α-naphthyl)vinyl, 2-(β-naphthyl)vinyl, 3-(α-naphthyl)-1-propenyl, 3-(α-naphthyl)-2-propenyl, 3-(β-naphthyl)-1-propenyl, 3-(β-naphthyl)-2-propenyl, 4-(α-naphthyl)-1-butenyl, 4-(α-naphthyl)-2-butenyl, 4-(α-naphthyl)-3-butenyl, 4-(β-naphthyl)-1-butenyl, 4-(β-naphthyl)-2-butenyl, 4-(β-naphthyl)-3-butenyl, and 3-(methoxyethoxyphenyl)butenyl can be typically mentioned. Species containing more carbon atoms are also included in the purview of the compounds of the invention.

The aryloxyalkenyl mentioned for R¹ is a group of about 8-20 carbon atoms. Thus, 1-phenoxyvinyl, 2-phenoxyvinyl, 2-phenoxy-1-propenyl, 2-phenoxy-2-propenyl, 3-phenoxy-1-propenyl, 3-phenoxy-2-propenyl, 3-phenoxy-1-butenyl, 3-phenoxy-2-butenyl, 3-phenoxy-3-butenyl, 4-phenoxy-1-butenyl, 4-phenoxy-2-butenyl, 4-phenoxy-3-butenyl, 2-(α-naphthyloxy)vinyl, 2-(β-naphthyloxy)vinyl, 3-(α-naphthyloxy)-1-propenyl, 3-(α-naphthyloxy)-2-propenyl, 3-(β-naphthyloxy)-1-propenyl, 3-(β-naphthyloxy)-2-propenyl, 4-(α-naphthyloxy)-1-butenyl, 4-(α-naphthyloxy)-2-butenyl, 4-(α-naphthyloxy)-3-butenyl, 4-(β-naphthyloxy)-1-butenyl, 4-(β-naphthyloxy)-2-butenyl, and 4-(β-naphthyloxy)-3-butenyl can be typically mentioned. Species containing more carbon atoms are also included in the purview of the compounds of the invention.

The aryloxyarylalkenyl mentioned for R¹ is a group of about 14-25 carbon atoms. Thus, 2-(phenoxyphenyl)vinyl, 3-(phenoxyphenyl)-1-propenyl, 3-(phenoxyphenyl)-2-propenyl, 4-(phenoxyphenyl)-2-butenyl, 4-(phenoxyphenyl)-3-butenyl, 3-(phenoxyphenyl)-2-butenyl, 5-(phenoxyphenyl)-4-pentenyl, 5-(phenoxyphenyl)-3-pentenyl, and 4-(phenoxyphenyl)-3-pentenyl can be typically mentioned. Groups containing more carbon atoms are also included in the purview of the compounds of the invention.

The alkylcarbamoylalkyl mentioned for R¹ is a group of about 3-20 carbon atoms, thus including but being not limited to methylcarbamoylmethyl, 1-methylcarbamoylethyl, 2-methylcarbamoylethyl, 1-methylcarbamoylpropyl, 2-methylcarbamoylpropyl, and 3-methylcarbamoylpropyl. Species containing more carbon atoms are also included in the purview of the compounds of the invention.

Where the substituent R¹ contains an aryl moiety, the aryl group may have a substituent or substituents selected from among halogen (fluoro, chloro, bromo, iodo), C1-4 alkyl, C1-4 alkoxy, and C2-10 alkoxycarbonyl.

The alkyl groups mentioned for R², R³, R⁴, and R⁵ may be the same or different and each may be a straight or branched-chain alkyl group of about 1-10 carbon atoms. Thus, lower alkyl groups such as methyl, ethyl, propyl, isopropyl, butyl, isobutyl, sec-butyl, tert-butyl, etc., intermediate alkyl groups such as pentyl, isopentyl, hexyl, isohexyl, etc., and higher alkyl groups such as decyl can be mentioned. Among them, lower alkyl groups such as methyl, ethyl, propyl, isopropyl, butyl, isobutyl, etc. are preferred.

The alkenyl groups for R², R³, R⁴, and R⁵ may be the same or different and each may be a straight or branched-chain group of about 2-20 carbon atoms. Thus, vinyl, 1-propenyl, 2-propenyl, isopropenyl, 1-butenyl, 2-butenyl, 3-butenyl, 2-methyl-1-propenyl, 2-methyl-2-propenyl, 1-methyl-1-propenyl, 1-methyl-2-propenyl, tert-butenyl, geranyl, and farnesyl can be typically mentioned. Species containing more carbon atoms are also included in the purview of the compounds of the invention.

The acyl groups for R², R³, R⁴, and R⁵ may be the same or different and each may be a linear or branched group of about 1-20 carbon atoms. Thus, formyl, acetyl, propionyl, isopropionyl, butyryl, isobutyryl, valeryl, isovaleryl, pivaloyl, hexanoyl, octanoyl, lauroyl, aroyl (benzoyl, toluoyl, α-naphthoyl, β-naphthoyl, 2-nicotinoyl, 3-nicotinoyl, 4-nicotinoyl, etc.), acryloyl, methacryloyl, oxalo, methoxalyl, ethoxalyl, cyclohexylcarbonyl, benzylcarbonyl, halo(fluoro, chloro, bromo, iodo)benzylcarbonyl, phenylbenzylcarbonyl, benzyloxycarbonyl, phenoxycarbonyl, phenylethylcarbonyl, cinnamoyl, methylmalonyl, ethylmalonyl, and propylmalonyl can be typically mentioned. Among the above groups, lower acyl groups such as acetyl, propionyl, isopropionyl, butyryl, isobutyryl, and valeryl are preferred. Species containing more carbon atoms are also included in the purview of the compounds of the invention.

For the purposes of the present invention, the compounds of the following general formula [II] are particularly preferred. 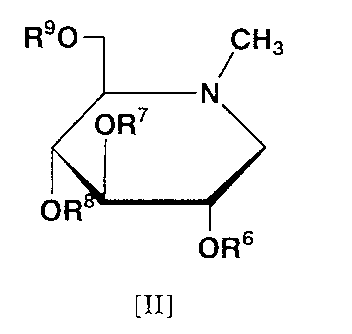 wherein R⁶, R⁷, R⁸, and R⁹ all represent the same C1-8 acyl group.

The acyl group mentioned for R⁶, R⁷, R⁸, and R⁹ includes but is not limited to formyl, acetyl, propionyl, isopropionyl, butyryl, isobutyryl, valeryl, isovaleryl, pivaloyl, hexanoyl, octanoyl, aroyl (benzoyl, toluoyl, etc.), acryloyl, methacryloyl, oxalo, methoxalyl, ethoxalyl, cyclohexylcarbonyl, halo(fluoro, chloro, bromo, iodo)benzylcarbonyl, benzyloxycarbonyl, phenoxycarbonyl, phenylethylcarbonyl, methylmalonyl, ethylmalonyl, and propylmalonyl. Particularly preferred is the compound in which all of R⁶, R⁷, R⁸, and R⁹ represent butyryl.

The physiologically acceptable salt mentioned herein before includes inorganic salts such as hydrochloride, nitrate, sulfate, hydrobromide, phosphate, etc., organic acid salts such as acetate, succinate, fumarate, maleate, malate, lactate, tartrate, methanesulfonate, tosylate, etc., quaternary salts such as potassium salt, sodium salt, ammonium salt.

The following is a partial list of specific compounds of the invention.
2,3,4,5-Tetra-O-acetylmoranoline
2,3,4,5-Tetra-O-butyrylmoranoline
2,3,4,5-Tetra-O-isobutyrylmoranoline
2,3,4,5-Tetra-O-benzoylmoranoline
2,3,4,5-Tetra-O-acetyl-N-methylmoranoline
2,3,4,5-Tetra-O-butyryl-N-methylmoranoline
2,3,4,5-Tetra-O-isobutyryl-N-methylmoranoline
2,3,4,5-Tetra-O-benzoyl-N-methylmoranoline
2,3,4,5-Tetra-O-acetyl-N-(n-butyl)moranoline
2,3,4,5-Tetra-O-butyryl-N-(n-butyl)moranoline
2,3,4,5-Tetra-O-isobutyryl-N-(n-butyl)moranoline
2,3,4,5-Tetra-O-benzoyl-N-(n-butyl)moranoline
2,3-Di-O-acetyl-N-(n-butyl)moranoline
2,3-Di-O-butyryl-N-(n-butyl)moranoline
2,3-Di-O-isobutyryl-N-(n-butyl)moranoline
2,3-Di-O-benzoyl-N-(n-butyl)moranoline
2,3,4,5-Tetra-O-acetyl-N-(5-methoxycarbonylpentyl) moranoline tosylate
2,3,4,5-Tetra-O-butyryl-N-(5-methoxycarbonylpentyl) moranoline tosylate
2,3,4,5-Tetra-O-isobutyryl-N-(5-methoxycarbonylpentyl) moranoline tosylate
2,3,4,5-Tetra-O-benzoyl-N-(5-methoxycarbonylpentyl) moranoline tosylate
2,3,4,5-Tetra-O-acetyl-N-(1-hydroxyethyl) moranoline tosylate
2,3,4,5-Tetra-O-butyryl-N-(1-hydroxyethyl) moranoline tosylate
2,3,4,5-Tetra-O-isobutyryl-N-(1-hydroxyethyl) moranoline tosylate
2,3,4,5-Tetra-O-benzoyl-N-(1-hydroxyethyl) moranoline tosylate
2,3,4,5-Tetra-O-acetyl-N-(2-hydroxyethyl) moranoline tosylate
2,3,4,5-Tetra-O-butyryl-N-(2-hydroxyethyl) moranoline tosylate
2,3,4,5-Tetra-O-isobutyryl-N-(2-hydroxyethyl) moranoline tosylate
2,3,4,5-Tetra-O-benzoyl-N-(2-hydroxyethyl) moranoline tosylate
2,3,4,5-Tetra-O-acetyl-N-(4-methoxycarbonylbutyl) moranoline tosylate
2,3,4,5-Tetra-O-butyryl-N-(4-methoxycarbonylbutyl) moranoline tosylate
2,3,4,5-Tetra-O-isobutyryl-N-(4-methoxycarbonylbutyl) moranoline tosylate
2,3,4,5-Tetra-O-benzoyl-N-(4-methoxycarbonylbutyl) moranoline tosylate
2,3,4,5-Tetra-O-acetyl-N-hexylmoranoline
2,3,4,5-Tetra-O-butyryl-N-hexylmoranoline
2,3,4,5-Tetra-O-isobutyryl-N-hexylmoranoline
2,3,4,5-Tetra-O-benzoyl-N-hexylmoranoline
2,3,4,5-Tetra-O-acetyl-N-hexylmoranoline tosylate
2,3,4,5-Tetra-O-butyryl-N-hexylmoranoline tosylate
2,3,4,5-Tetra-O-isobutyryl-N-hexylmoranoline tosylate
2,3,4,5-Tetra-O-benzoyl-N-hexylmoranoline tosylate
2,3-Di-O-acetyl-N-hexylmoranoline
2,3-Di-O-butyryl-N-hexylmoranoline
2,3-Di-O-isobutyryl-N-hexylmoranoline
2,3-Di-O-benzoyl-N-hexylmoranoline
2,3,4,5-Tetra-O-acetyl-N-isoprenylmoranoline
2,3,4,5-Tetra-O-butyryl-N-isoprenylmoranoline
2,3,4,5-Tetra-O-isobutyryl-N-isoprenylmoranoline
2,3,4,5-Tetra-O-benzoyl-N-isoprenylmoranoline
2,3,4,5-Tetra-O-acetyl-N-(2-hydroxydecyl)moranoline
2,3,4,5-Tetra-O-butyryl-N-(2-hydroxydecyl)moranoline
2,3,4,5-Tetra-O-isobutyryl-N-(2-hydroxydecyl)moranoline
2,3,4,5-Tetra-O-benzoyl-N-(2-hydroxydecyl)moranoline
2,3,4,5-Tetra-O-acetyl-N-(2-hydroxydecyl)moranoline tosylate
2,3,4,5-Tetra-O-butyryl-N-(2-hydroxydecyl)moranoline tosylate
2,3,4,5-Tetra-O-isobutyryl-N-(2-hydroxydecyl)moranoline tosylate
2,3,4,5-Tetra-O-benzoyl-N-(2-hydroxydecyl)moranoline tosylate
2,3,4,5-Tetra-O-acetyl-N-(10-carboxydecyl)moranoline
2,3,4,5-Tetra-O-butyryl-N-(10-carboxydecyl)moranoline
2,3,4,5-Tetra-O-isobutyryl-N-(10-carboxydecyl)moranoline
2,3,4,5-Tetra-O-benzoyl-N-(10-carboxydecyl)moranoline
2,3,4,5-Tetra-O-acetyl-N-(10-carboxydecyl)moranoline sodium salt
2,3,4,5-Tetra-O-butyryl-N-(10-carboxydecyl)moranoline sodium salt
2,3,4,5-Tetra-O-isobutyryl-N-(10-carboxydecyl)moranoline sodium salt
2,3,4,5-Tetra-O-benzoyl-N-(10-carboxydecyl)moranoline sodium salt
2,3,4,5-Tetra-O-acetyl-N-(3-phenylpropyl)moranoline
2,3,4,5-Tetra-O-butyryl-N-(3-phenylpropyl)moranoline
2,3,4,5-Tetra-O-isobutyryl-N-(3-phenylpropyl)moranoline
2,3,4,5-Tetra-O-benzoyl-N-(3-phenylpropyl)moranoline
2,3,4,5-Tetra-O-acetyl-N-(3-phenylpropyl)moranoline tosylate
2,3,4,5-Tetra-O-butyryl-N-(3-phenylpropyl)moranoline tosylate
2,3,4,5-Tetra-O-isobutyryl-N-(3-phenylpropyl)moranoline tosylate
2,3,4,5-Tetra-O-benzoyl-N-(3-phenylpropyl)moranoline tosylate
2,3,4,5-Tetra-O-acetyl-N-benzylmoranoline
2,3,4,5-Tetra-O-butyryl-N-benzylmoranoline
2,3,4,5-Tetra-O-isobutyryl-N-benzylmoranoline
2,3,4,5-Tetra-O-benzoyl-N-benzylmoranoline
2,3,4,5-Tetra-O-acetyl-N-cinnamoylmoranoline
2,3,4,5-Tetra-O-butyryl-N-cinnamoylmoranoline
2,3,4,5-Tetra-O-isobutyryl-N-cinnamoylmoranoline
2,3,4,5-Tetra-O-benzoyl-N-cinnamoylmoranoline
2,3,4,5-Tetra-O-acetyl-N-cinnamoylmoranoline hydrochloride
2,3,4,5-Tetra-O-butyryl-N-cinnamoylmoranoline hydrochloride
2,3,4,5-Tetra-O-isobutyryl-N-cinnamoylmoranoline hydrochloride
2,3,4,5-Tetra-O-benzoyl-N-cinnamoylmoranoline hydrochloride
2,3,4,5-Tetra-O-acetyl-N-(2-phenylethyl)carbonylmoranoline
2,3,4,5-Tetra-O-butyryl-N-(2-phenylethyl)carbonylmoranoline
2,3,4,5-Tetra-O-isobutyryl-N-(2-phenylethyl)carbonylmoranoline
2,3,4,5-Tetra-O-benzoyl-N-(2-phenylethyl)carbonylmoranoline
2,3,4,5-Tetra-O-acetyl-N-(3-phenylbutyl)moranoline
2,3,4,5-Tetra-O-butyryl-N-(3-phenylbutyl)moranoline
2,3,4,5-Tetra-O-isobutyryl-N-(3-phenylbutyl)moranoline
2,3,4,5-Tetra-O-benzoyl-N-(3-phenylbutyl)moranoline
2,3,4,5-Tetra-O-acetyl-N-(3-phenylbutyl)moranoline tosylate
2,3,4,5-Tetra-O-butyryl-N-(3-phenylbutyl)moranoline tosylate
2,3,4,5-Tetra-O-isobutyryl-N-(3-phenylbutyl)moranoline ztosylate
2,3,4,5-Tetra-O-benzoyl-N-(3-phenylbutyl)moranoline tosylate
2,3,4,5-Tetra-O-acetyl-N-(4-phenylbutyl)moranoline
2,3,4,5-Tetra-O-butyryl-N-(4-phenylbutyl)moranoline
2,3,4,5-Tetra-O-isobutyryl-N-(4-phenylbutyl)moranoline
2,3,4,5-Tetra-O-benzoyl-N-(4-phenylbutyl)moranoline
2,3,4,5-Tetra-O-acetyl-N-(4-phenylbutyl)moranoline tosylate
2,3,4,5-Tetra-O-butyryl-N-(4-phenylbutyl)moranoline tosylate
2,3,4,5-Tetra-O-isobutyryl-N-(4-phenylbutyl)moranoline tosylate
2,3,4,5-Tetra-O-benzoyl-N-(4-phenylbutyl)moranoline tosylate
2,3,4,5-Tetra-O-acetyl-N-(2-phenoxyethyl)moranoline
2,3,4,5-Tetra-O-butyryl-N-(2-phenoxyethyl)moranoline
2,3,4,5-Tetra-O-isobutyryl-N-(2-phenoxyethyl)moranoline
2,3,4,5-Tetra-O-benzoyl-N-(2-phenoxyethyl)moranoline
2,3,4,5-Tetra-O-acetyl-N-(3-phenoxypropyl)moranoline
2,3,4,5-Tetra-O-butyryl-N-(3-phenoxypropyl)moranoline
2,3,4,5-Tetra-O-isobutyryl-N-(3-phenoxypropyl)moranoline
2,3,4,5-Tetra-O-benzoyl-N-(3-phenoxypropyl)moranoline
2,3,4,5-Tetra-O-acetyl-N-(3-phenoxypropyl)moranoline tosylate
2,3,4,5-Tetra-O-butyryl-N-(3-phenoxypropyl)moranoline tosylate
2,3,4,5-Tetra-O-isobutyryl-N-(3-phenoxypropyl)moranoline tosylate
2,3,4,5-Tetra-O-benzoyl-N-(3-phenoxypropyl)moranoline tosylate
2,3,4,5-Tetra-O-acetyl-N-(5-phenylpentyl)moranoline
2,3,4,5-Tetra-O-butyryl-N-(5-phenylpentyl)moranoline
2,3,4,5-Tetra-O-isobutyryl-N-(5-phenylpentyl)moranoline
2,3,4,5-Tetra-O-benzoyl-N-(5-phenylpentyl)moranoline
2,3,4,5-Tetra-O-acetyl-N-(5-phenylpentyl)moranoline tosylate
2,3,4,5-Tetra-O-butyryl-N-(5-phenylpentyl)moranoline tosylate
2,3,4,5-Tetra-O-isobutyryl-N-(5-phenylpentyl)moranoline tosylate
2,3,4,5-Tetra-O-benzoyl-N-(5-phenylpentyl)moranoline tosylate
2,3,4,5-Tetra-O-acetyl-N-(2-cyclopentylethyl)moranoline
2,3,4,5-Tetra-O-butyryl-N-(2-cyclopentylethyl)moranoline
2,3,4,5-Tetra-O-isobutyryl-N-(2-cyclopentylethyl) moranoline
2,3,4,5-Tetra-O-benzoyl-N-(2-cyclopentylethyl)moranoline
2,3,4,5-Tetra-O-acetyl-N-(2-cyclopentylethyl)moranoline tosylate
2,3,4,5-Tetra-O-butyryl-N-(2-cyclopentylethyl)moranoline tosylate
2,3,4,5-Tetra-O-isobutyryl-N-(2-cyclopentylethyl) moranoline tosylate
2,3,4,5-Tetra-O-benzoyl-N-(2-cyclopentylethyl)moranoline tosylate
2,3,4,5-Tetra-O-acetyl-N-[3-(3-(2-methoxyethoxy)phenyl)-2-butenyl] moranoline
2,3,4,5-Tetra-O-butyryl-N-[3-(3-(2-methoxyethoxy)phenyl)-2-butenyl]moranoline
2,3,4,5-Tetra-O-isobutyryl-N-[3-(3-(2-methoxyethoxy) phenyl)-2-butenyl] moranoline
2,3,4,5-Tetra-O-benzoyl-N-[3-(3-(2-methoxyethoxy) phenyl)-2-butenyl]moranoline
2,3,4,5-Tetra-O-acetyl-N,N-dimethylmoranoline ammonium iodide
2,3,4,5-Tetra-O-butyryl-N,N-dimethylmoranoline ammonium iodide
2,3,4,5-Tetra-O-isobutyryl-N,N-dimethylmoranoline ammonium iodide
2,3,4,5-Tetra-O-benzoyl-N,N-dimethylmoranoline ammonium iodide
2,3,4,5-Tetra-O-acetyl-N-ethylmoranoline
2,3,4,5-Tetra-O-butyryl-N-ethylmoranoline
2,3,4,5-Tetra-O-isobutyryl-N-ethylmoranoline
2,3,4,5-Tetra-O-benzoyl-N-ethylmoranoline
2,3,4,5-Tetra-O-acetyl-N-geranylmoranoline
2,3,4,5-Tetra-O-butyryl-N-geranylmoranoline
2,3,4,5-Tetra-O-isobutyryl-N-geranylmoranoline
2,3,4,5-Tetra-O-benzoyl-N-geranylmoranoline
2,3,4,5-Tetra-O-acetyl-N-geranylmoranoline tosylate
2,3,4,5-Tetra-O-butyryl-N-geranylmoranoline tosylate
2,3,4,5-Tetra-O-isobutyryl-N-geranylmoranoline tosylate
2,3,4,5-Tetra-O-benzoyl-N-geranylmoranoline tosylate
2,3,4,5-Tetra-O-acetyl-N-(2-hydroxy-3-phenoxypropyl) moranoline
2,3,4,5-Tetra-O-butyryl-N-(2-hydroxy-3-phenoxypropyl) moranoline
2,3,4,5-Tetra-O-isobutyryl-N-(2-hydroxy-3-phenoxypropyl) moranoline
2,3,4,5-Tetra-O-benzoyl-N-(2-hydroxy-3-phenoxypropyl) moranoline
2,3,4,5-Tetra-O-acetyl-N-(2-hydroxy-3-phenoxypropyl) moranoline tosylate
2,3,4,5-Tetra-O-butyryl-N-(2-hydroxy-3-phenoxypropyl) moranoline tosylate
2,3,4,5-Tetra-O-isobutyryl-N-(2-hydroxy-3-phenoxypropyl)moranolinetosylate
2,3,4,5-Tetra-O-benzoyl-N-(2-hydroxy-3-phenoxypropyl) moranoline tosylate
2,3,4,5-Tetra-O-acetyl-N-farnesylmoranoline
2,3,4,5-Tetra-O-butyryl-N-farnesylmoranoline
2,3,4,5-Tetra-O-isobutyryl-N-farnesylmoranoline
2,3,4,5-Tetra-O-benzoyl-N-farnesylmoranoline
2,3,4,5-Tetra-O-acetyl-N-farnesylmoranolinium tosylate
2,3,4,5-Tetra-O-butyryl-N-farnesylmoranolinium tosylate
2,3,4,5-Tetra-O-isobutyryl-N-farnesylmoranolinium tosylate
2,3,4,5-Tetra-O-benzoyl-N-farnesylmoranolinium tosylate
2,3,4,5-Tetra-O-acetyl-N-[10-(N-methylcarbamoyl)decyl] moranoline
2,3,4,5-Tetra-O-butyryl-N-[10-(N-methylcarbamoyl)decyl] moranoline
2,3,4,5-Tetra-O-isobutyryl-N-[10-(N-methylcarbamoyl) decyl] moranoline
2,3,4,5-Tetra-O-benzoyl-N-[10-(N-methylcarbamoyl)decyl] moranoline
2,3,4,5-Tetra-O-acetyl-N-(4-phenyl-3-butenyl)moranoline
2,3,4,5-Tetra-O-butyryl-N-(4-phenyl-3-butenyl)moranoline
2,3,4,5-Tetra-O-isobutyryl-N-(4-phenyl-3-butenyl) moranoline
2,3,4,5-Tetra-O-benzoyl-N-(4-phenyl-3-butenyl)moranoline tosylate
2,3,4,5-Tetra-O-acetyl-N-(4-phenyl-3-butenyl)moranoline tosylate
2,3,4,5-Tetra-O-butyryl-N-(4-phenyl-3-butenyl)moranoline tosylate
2,3,4,5-Tetra-O-isobutyryl-N-(4-phenyl-3-butenyl) moranoline tosylate
2,3,4,5-Tetra-O-benzoyl-N-(4-phenyl-3-butenyl)moranoline tosylate
2,3,4,5-Tetra-O-acetyl-N-(3-phenyl-2-methyl-2-propenyl) moranoline
2,3,4,5-Tetra-O-butyryl-N-(3-phenyl-2-methyl-2-propenyl) moranoline
2,3,4,5-Tetra-O-isobutyryl-N-(3-phenyl-2-methyl-2-propenyl) moranoline
2,3,4,5-Tetra-O-benzoyl-N-(3-phenyl-2-methyl-2-propenyl) moranoline
2,3,4,5-Tetra-O-acetyl-N-[3-(o-chlorophenoxy)propyl] moranoline
2,3,4,5-Tetra-O-butyryl-N-[3-(o-chlorophenoxy)propyl] moranoline
2,3,4,5-Tetra-O-isobutyryl-N-[3-(o-chlorophenoxy)propyl] moranoline
2,3,4,5-Tetra-O-benzoyl-N-[3-(o-chlorophenoxy)propyl] moranoline
2,3,4,5-Tetra-O-acetyl-N-(γ-methyl-p-bromocinnamoyl) moranoline
2,3,4,5-Tetra-O-butyryl-N-(γ-methyl-p-bromocinnamoyl) moranoline
2,3,4,5-Tetra-O-isobutyryl-N-(γ-methyl-p-bromocinnamoyl) moranoline
2,3,4,5-Tetra-O-benzoyl-N-(γ-methyl-p-bromocinnamoyl) moranoline
2,3,4,5-Tetra-O-acetyl-N-[4-(3-fluoro-4-methylphenyl) butyl]moranoline
2,3,4,5-Tetra-O-butyryl-N-[4-(3-fluoro-4-methylphenyl) butyl]moranoline
2,3,4,5-Tetra-O-isobutyryl-N-[4-(3-fluoro-4-methylphenyl) butyl]moranoline
2,3,4,5-Tetra-O-benzoyl-N-[4-(3-fluoro-4-methylphenyl) butyl]moranoline
2,3,4,5-Tetra-O-acetyl-N-[4-(3-fluoro-4-methylphenyl) butyl]moranoline
2,3,4,5-Tetra-O-butyryl-N-[4-(3-fluoro-4-methylphenyl) butyl]moranoline
2,3,4,5-Tetra-O-isobutyryl-N-[4-(3-fluoro-4-methylphenyl) butyl]moranoline
2,3,4,5-Tetra-O-benzoyl-N-[4-(3-fluoro-4-methylphenyl) butyl]moranoline
2,3,4,5-Tetra-O-acetyl-N-(p-ethoxycinnamoyl)moranoline
2,3,4,5-Tetra-O-butyryl-N-(p-ethoxycinnamoyl)moranoline
2,3,4,5-Tetra-O-isobutyryl-N-(p-ethoxycinnamoyl)moranoline
2,3,4,5-Tetra-O-benzoyl-N-(p-ethoxycinnamoyl)moranoline
2,3,4,5-Tetra-O-acetyl-N-(p-isopropoxycinnamoyl)moranoline
2,3,4,5-Tetra-O-butyryl-N-(p-isopropoxycinnamoyl)moranoline
2,3,4,5-Tetra-O-isobutyryl-N-(p-isopropoxycinnamoyl)moranoline
2,3,4,5-Tetra-O-benzoyl-N-(p-isopropoxycinnamoyl)moranoline
2,3,4,5-Tetra-O-acetyl-N-(γ-methyl-m-methylcinnamoyl) moranoline
2,3,4,5-Tetra-O-butyryl-N-(γ-methyl-m-methylcinnamoyl) moranoline
2,3,4,5-Tetra-O-isobutyryl-N-(γ-methyl-m-methylcinnamoyl) moranoline
2,3,4,5-Tetra-O-benzoyl-N-(γ-methyl-m-methylcinnamoyl) moranoline
2,3,4,5-Tetra-O-acetyl-N-[4-(m-methoxyphenyl)-3-pentenyl] moranoline
2,3,4,5-Tetra-O-butyryl-N-[4-(m-methoxyphenyl)-3-pentenyl] moranoline
2,3,4,5-Tetra-O-isobutyryl-N-[4-(m-methoxyphenyl)-3-pentenyl] moranoline
2,3,4,5-Tetra-O-benzoyl-N-[4-(m-methoxyphenyl)-3-pentenyl] moranoline
2,3,4,5-Tetra-O-aceryl-N-[2-(p-ethoxycarbonylphenoxy)ethyl] moranoline
2,3,4,5-Tetra-O-butyryl-N-[2-(p-ethoxycarbonylphenoxy)ethyl] moranoline
2,3,4,5-Tetra-O-isobutyryl-N-[2-(p-ethoxycarbonylphenoxy)ethyl] moranoline
2,3,4,5-Tetra-O-benzoyl-N-[2-(p-ethoxycarbonylphenoxy)ethyl] moranoline
2,3,4,5-Tetra-O-acetyl-N-nonylmoranoline
2,3,4,5-Tetra-O-butyryl-N-nonylmoranoline
2,3,4,5-Tetra-O-isobutyryl-N-nonylmoranoline
2,3,4,5-Tetra-O-benzoyl-N-nonylmoranoline
2,3,4,5-Tetra-O-acetyl-N-benzyloxycarbonylmoranoline
2,3,4,5-Tetra-O-butyryl-N-benzyloxycarbonylmoranoline
2,3,4,5-Tetra-O-isobutyryl-N-benzyloxycarbonylmoranoline
2,3,4,5-Tetra-O-benzoyl-N-benzyloxycarbonylmoranoline
2,3,4,5-Tetra-O-acetyl-N-phenoxycarbonylmoranoline
2,3,4,5-Tetra-O-butyryl-N-phenoxycarbonylmoranoline
2,3,4,5-Tetra-O-isobutyryl-N-phenoxycarbonylmoranoline
2,3,4,5-Tetra-O-benzoyl-N-phenoxycarbonylmoranoline
2,3,4,5-Tetra-O-acetyl-N-benzylcarbonylmoranoline
2,3,4,5-Tetra-O-butyryl-N-benzylcarbonylmoranoline
2,3,4,5-Tetra-O-isobutyryl-N-benzylcarbonylmoranoline
2,3,4,5-Tetra-O-benzoyl-N-benzylcarbonylmoranoline
2,3,4,5-Tetra-O-acetyl-N-benzoylcarbonylmoranoline
2,3,4,5-Tetra-O-butyryl-N-benzoylcarbonylmoranoline
2,3,4,5-Tetra-O-isobutyryl-N-benzoylcarbonylmoranoline
2,3,4,5-Tetra-O-benzoyl-N-benzoylcarbonylmoranoline
2,3,4,5-Tetra-O-acetyl-N-(ethylmalonyl)moranoline
2,3,4,5-Tetra-O-butyryl-N-(ethylmalonyl)moranoline
2,3,4,5-Tetra-O-isobutyryl-N-(ethylmalonyl)moranoline
2,3,4,5-Tetra-O-benzoyl-N-(ethylmalonyl)moranoline
2,3,4,5-Tetra-O-acetyl-N-(2-methylpentyl)moranoline
2,3,4,5-Tetra-O-butyryl-N-(2-methylpentyl)moranoline
2,3,4,5-Tetra-O-isobutyryl-N-(2-methylpentyl)moranoline
2,3,4,5-Tetra-O-benzoyl-N-(2-methylpentyl)moranoline
2,3,4,5-Tetra-O-acetyl-N-(2-ethylbutyl)moranoline
2,3,4,5-Tetra-O-butyryl-N-(2-ethylbutyl)moranoline
2,3,4,5-Tetra-O-isobutyryl-N-(2-ethylbutyl)moranoline
2,3,4,5-Tetra-O-benzoyl-N-(2-ethylbutyl)moranoline
2,3,4,5-Tetra-O-acetyl-N-(4-chlorobenzylcarbonyl)moranoline
2,3,4,5-Tetra-O-butyryl-N-(4-chlorobenzylcarbonyl)moranoline
2,3,4,5-Tetra-O-isobutyryl-N-(4-chlorobenzylcarbonyl)moranoline
2,3,4,5-Tetra-O-benzoyl-N-(4-chlorobenzylcarbonyl)moranoline
2,3,4,5-Tetra-O-acetyl-N-(4-phenylbenzylcarbonyl)moranoline
2,3,4,5-Tetra-O-butyryl-N-(4-phenylbenzylcarbonyl)moranoline
2,3,4,5-Tetra-O-isobutyryl-N-(4-phenylbenzylcarbonyl)moranoline
2,3,4,5-Tetra-O-benzoyl-N-(4-phenylbenzylcarbonyl)moranoline
2,3,4,5-Tetra-O-acetyl-N-nicotinoylmoranoline
2,3,4,5-Tetra-O-butyryl-N-nicotinoylmoranoline
2,3,4,5-Tetra-O-isobutyryl-N-nicotinoylmoranoline
2,3,4,5-Tetra-O-benzoyl-N-nicotinoylmoranoline
2,3-Di-O-acetyl-4,6-O-(R)-benzylidene-N-butylmoranoline
2,3-Di-O-butyryl-4,6-O-(R)-benzylidene-N-butylmoranoline
2,3-Di-O-acetyl-4,6-O-(R)-benzylidene-N-hexylmoranoline
2,3,4,5-Tetra-O-acetyl-N-[4-(p-phenoxyphenyl)-3-pentenyl]moranoline
2,3,4,5-Tetra-O-butyryl-N-[4-(p-phenoxyphenyl)-3-pentenyl]moranoline
2,3,4,5-Tetra-O-isobutyryl-N-[4-(p-phenoxyphenyl)-3-pentenyl]moranoline
2,3,4,5-Tetra-O-benzoyl-N-[4-(p-phenoxyphenyl)-3-pentenyl] moranoline

The compounds of the invention include not only the compounds listed above but also the 2,3,4,5-tetra-O-acetyl, 2,3,4,5-tetra-O-butyryl, 2,3,4,5-tetra-O-isobutyryl, 2,3,4,5-tetra-O-benzoyl, and other derivatives of the following compounds.
N-isobutylmoranoline tosylate
N-(2-hydroxy)ethylmoranoline tosylate
N-aminomornoline hydrobromide
N-(2-methoxyethyl)moranoline tosylate
N-[2-(2-methoxyethoxy)ethyl]moranoline tosylate
N-decylmoranoline tosylate
N-(2-hydroxyhexadecyl)moranoline toyslate
N-[2-hydroxy-3-(p-tolyloxy)propyl]moranoline tosylate
N-[2-hydroxy-3-(p-methoxyphenyloxy)propyl]moranoline tosylate
N-[2-hydroxy-3-(p-chlorophenyloxy)propyl]moranoline tosylate
N-(3-carbamoylpropyl)moranoline tosylate
N-nonylmoranoline tosylate
N-undecylmoranoline tosylate
N-(2-hydroxytetradecyl)moranoline tosylate
N-(4,4-diphenyl-3-butenyl)moranoline
N-(5-carboxypentyl)moranoline
N-(γ-methyl-p-chlorocinnamoyl)moranoline
N-(γ-methyl-p-methylcinnamoyl)moranoline
N-[4-(p-chlorophenyl)-3-pentenyl]moranoline
N-[4-(m-chlorophenyl)-3-pentenyl]moranoline
N-[4-(o-chlorophenyl)-3-pentenyl]moranoline
N-[4-(p-phenoxyphenyl)-3-pentenyl]moranoline
N-[4-(p-ethoxyphenyl)-3-pentenyl]moranoline
N-(p-methoxycinnamoyl)moranoline
N-[3-(3-chlorophenyl)-2-butenyl]moranoline
N-[4-(4-chlorophenyl)-3-butenyl]moranoline
N-(4-carboxycinnamoyl)moranoline hydrochloride
N-(3-carboxy-2-propenyl)moranoline
N-[m-(2-triethylammonioethoxy)cinnamoyl]moranoline dipicrate
N-isopropylmoranoline
N-[p-(2-trimethylammonioethoxy)cinnamoyl]moranoline hydrochloride

The compounds of the invention can be synthesized by the known production technology such as the process disclosed in Japanese Patent Application Kokai H 2-172975. For example, the objective compound can be produced by subjecting moranoline to N-alkylation, N-acylation (JP Application Kokai H 1-250350), or other reaction in the conventional manner, either preceded or followed by O-alkylation, O-acylation, or other reaction of the free hydroxyl group.

The compounds of the invention have α₂-PI inhibitory activity and urokinase secretion-promoting activity and is of value as a therapeutic or prophylactic drug for thrombosis.

It is well documented that the toxic potential of the compounds of the invention are very low.

For administration as a drug, the compounds of the invention can be used in combination or admixture with a concomitant drug. The compounds of the invention can be administered to animals inclusive of man, either as it is or in the form of a pharmaceutical composition containing 10%-95%, preferably 50%-90%, of the compounds of the invention in a medicinally acceptable, nontoxic and inert carrier.

The carrier that can be used includes one or more members of solid, semi-solid or liquid diluents, fillers, and other auxiliary agents. The pharmaceutical composition is preferably administered in unit dosage forms and by the oral, parenteral, local (transdermal etc.), or rectal route. Of course, dosage forms tailored to the respective routes of administration are employed. Oral or parenteral administration, for instance, is preferred, and oral administration is particularly preferred.

The dosage for the therapy or prophylaxis of thrombosis is preferably selected according to patient factors such as age, body weight, etc., route of administration, nature and severity of illness, etc. Usually, however, the range of 0.1-10 g/day/man, preferably 1-3 g/day/man, as the active compounds of the invention are recommended for an average adult. There may be cases in which lower doses are sufficient, while larger doses may be a judicious choice in other cases. The above daily dosage can be administered in 2-3 divided doses.

Oral administration can be carried out using solid or liquid unit dosage forms such as fine powders, capsules, tablets, dragees, granules, powders, suspensions, solutions, syrups, drops, sublingual tablets, etc.

The fine powders can be manufactured by pulverizing the active compound to a suitable particle size. The powders can be manufactured by pulverizing the active compound to a suitable particle size and mixing it with a similarly pulverized pharmaceutical carrier such as edible carbohydrates, e.g. starch, mannitol, etc., and other suitable materials. Where necessary, a flavorant, preservative, dispersant, coloring agent, perfume, etc. can also be added.

The capsules can be manufactured by filling gelatin or other capsule shells with the fine powder or powder prepared as above or granules prepared by the procedure described for tablets. Lubricants and/or fluidizing agents, such as colloidal silica, talc, magnesium stearate, calcium stearate, and solid polyethylene glycol, may be added in pulverised form prior to the filling operation described above. Disintegrators and solubilizers, such as carboxymethylcellulose, carboxymethylcellulose calcium, low-substitution-degree hydroxypropylcellulose, croscarmellose sodium, carboxymethylstarch sodium, calcium carbonate, and sodium carbonate can also be added, in which case the efficacy of the drug after ingestion of the capsules may be enhanced.

The finely pulverised compounds of the invention can be suspended and dispersed in vegetable oil, polyethylene glycol, glycerin, or a surfactant and packaged in a gelatin sheet to provide soft capsules. The tablets can be manufactured by preparing a powdery mixture of the compound with an excipient, processing it into granules or slags, adding a disintegrator and/or a lubricant, and compressing the mixture. The powdery mixture can be prepared by mixing an adequately pulverized powder of the active compound with any of said diluents or carriers. Where necessary, binders (e.g. carboxymethylcellulose sodium, methylcellulose, hydroxypropylmethylcellulose, gelatin, polyvinylpyrrolidone, polyvinyl alcohol, etc.), dissolution retardants (e.g. paraffin), reabsorption agents (e.g. a quaternary salt), and/or adsorbents (e.g. bentonite, kaolin, dicalcium phosphate, etc.) can also be added. The powdery mixture can be made into granules by wetting it with a binder, such as a syrup, a starch paste, gum arabic, a cellulose solution or a polymer solution, stirring the wet powder well, drying it, and pulverizing the same. Instead of converting the powder to granules in the above manner, the powder may be compressed with a tablet machine and the resulting crude slags be comminuted into granules.

The granules thus prepared can be protected against interadhesion by adding a lubricant such as stearic acid, a salt of stearic acid, talc, mineral oil or the like. The thus-lubricated composition is then compressed. The resulting uncoated tablets can be coated with a film coating or sugar coating composition.

As an alternative, the active compound can be directly mixed with a free-flowing inert carrier without being subjected to the above-mentioned granulation or slagging procedure and the mixture be directly compressed. A transparent or translucent protective coating capable of yielding a hermetic film of shellac or the like, a sugar or polymer coating, or a glaze wax coating, for instance, can also be applied.

Other dosage forms for oral administration, such as solution, syrup, or elixir, can also be provided in unit dosage forms each containing a predetermined amount of the drug. Syrups are manufactured by dissolving the active compound in a suitable flavored aqueous medium, while elixirs are manufactured using a nontoxic alcoholic vehicle. Suspensions are prepared by dispersing the active compound in a nontoxic vehicle. Where necessary, solubilizers and emulsifiers (e.g. ethoxylated isostearyl alcohol, polyoxyethylene sorbitol ester, etc.) as well as preservatives and flavorants (e.g. peppermint oil, saccharin, etc.) can also be added.

If necessary, unit dosage formulations for oral administration can be microencapsulated. Such formulations can also be coated or embedded in a polymer or wax matrix for prolonged action or sustained release.

Parenteral administration can be carried out using liquid unit dosage forms, e.g. solution or suspension, for subcutaneous, intramuscular or intravenous injection. Such dosage forms can be manufactured by suspending or dissolving a predetermined amount of the active compound in an injectable nontoxic liquid vehicle, e.g. an aqueous medium or an oily medium, and sterilizing the resulting suspension or solution. To make an injection isotonic, a nontoxic salt or a solution thereof can be added. Moreover, stabilizers, preservatives, emulsifiers, and other additives can also be employed.

For rectal administration, use can be made of suppositories manufactured by dissolving or suspending the active compound in a water-soluble or water-insoluble solid medium, e.g. polyethylene glycol, cacao butter, a semi-synthetic oleaginous base (e.g. Witepsol™), a higher fatty acid ester (e.g. myristyl palmitate) or a mixture thereof.

The dosage form of the present invention may contain, in addition to the active compounds of the invention, such other active ingredients as UK, streptokinase, and TPA.

### BEST MODE OF PRACTICING THE INVENTION

The present invention is now described in further detail with reference to some representative examples.

### Production Example 1

### Synthesis of 2,3,4,5-tetra-O-butyryl-N-methyl- moranoline (hereinafter referred to as compound A)

N-methylmoranoline (hereinafter referred to as compound B) (50 g, 0.282 mol) and 4-dimethylaminopyridine (3.45 g, 0.028 mol) were dissolved in pyridine (450 ml), and while the solution was stirred at room temperature, butyric anhydride (187.49 g, 1.185 mol) was gradually added dropwise. After the temperature rose to 50°C due to the heat evolved, the dropwise addition was further continued at 50°C with occasional cooling in a water bath. After completion of dropwise addition, the reaction was further conducted at 50°C with stirring for 4.5 hr. This reaction mixture was added to 3 L of iced water and extracted with n-hexane. The organic layer was washed with saturated aqueous sodium hydrogen carbonate solution and water in that order, dried, and concentrated under reduced pressure to recover 131.2 g of yellow oil. This oil was purified by silica gel column chromatography (eluent: n-hexane-ethyl acetate = 85:15) to provide 115.7 g (yield 89%) of the title compound.

| Elemental analysis | | | |
|---|---|---|---|
| Calcd. | C: 60.38 | H: 8.59 | N: 3.06 |
| Found. | C: 60.09 | H: 8.64 | N: 2.91 |

NMR (CDCl₃)
δ: 0.83-1.03 (m, 12H), 1.46-1.75 (m, 8H), 2.14-2.40 (m, 9H), 2.36 (s, 3H), 3.07-3.20 (m, 1H), 4.02-4.28 (m, 2H), 4.93-5.22 (m, 3H)
Mass spectrum
m/z: 458 (M+1), 370, 110

### Test Example 1 Assay of α₂-PI activity

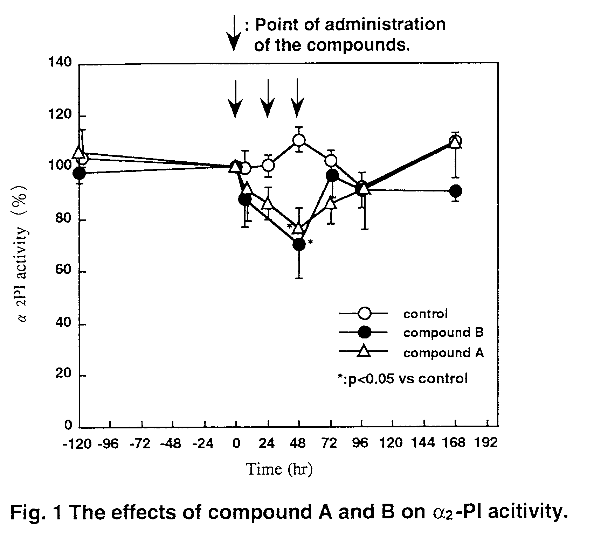

The drugs were administered to groups of 4 beagle dogs (22-23 months old, body weights 9-11 kg) and the blood was serially sampled (-120, 0, 6, 24, 48, 72, 96, and 168 hr) for assay of α₂-PI activity. One capsule of either compound A or compound B was administered orally once a day for 3 days (at 0, 24, and 48 hr) [116 mg/kg compound A or 300 mg/kg compound B (equimolar to compound A)]. Blood sampling was carried out by drawing 9 volumes of blood into a vacuum blood sampling tube containing 1 volume of 3.8% sodium citrate and, after gentle mixing, each blood sample was centrifuged (3,000 rpm x 10 min.) to separate the plasma. The plasma α₂-PI activity was assayed with Test Team APL 2 Kit using the cleavage of the synthetic chromogenic substrate S-2251 as an indicator by plasmin. The results are shown in Fig. 1.

It is apparent from Fig. 1 that compound A, i.e. the compound of the invention, decreased α₂-PI activity significantly and its α₂-PI decreasing activity was comparable with that of compound B which is a known α₂-PI antagonist.

### Test Example 2 Fibrinolytic action

The plasma obtained 48 hr after drug administration in Test Example 1 was used in this test.

To 0.35 ml of plasma was added 40 µl ¹²⁵I-fibrinogen (185 kBq/ml) and the mixture was dispensed to 3 test tubes, 0.1 ml per tube. To each of the test tubes was added 10 µl each of 100 µU/ml thrombin solution and 0.5 M calcium chloride solution and, after stirring, each tube was incubated at 37°C for 30 min to prepare a fibrin clot. Then, 2% albumin-saline containing 0, 50 or 100 U of urokinase was added to the tubes, 1 ml per tube. The tubes were incubated at 37°C and at 0, 3, 6, 9, 21 and 24 hr of incubation, 50µl of the supernatant was taken in a RIA tube and using a γ-counter (Packard Japan, A5424), the amount of the ¹²⁵I-fibrin degradated product was determined. The results after 24 hr are shown in Table 1.

**Table 1**

| Fibrinolysis (%) : (24 hr after addition of Urokinase) | | | | |
|---|---|---|---|---|
| Urokinase (unit) | N | Control | Comound B (116mg/kg) | Compound A (300mg/kg) |
| 0U | 4 | 100 ± 8 | 172 ± 56 | 143 ± 27 |
| 50U | 4 | 187 ± 14 | 213 ± 27 | 278 ± 31 |
| 100U | 4 | 193 ± 14 | 340 ± 49 | 342 ± 30 |
| Values are represented as mean + S.E. * : p<0.05 vs. Control (Dunnett's method) | | | | |

It is apparent from Table 1 that compound A, i.e. the compound of the invention, significantly accelerated the fibrinolytic activity of the urokinase addition level of 100 U, thus emulating the fibrinolytic activity of compound B which is a known fibrinolytic compound.

### Test Example 3 Observation for evaluation of diarrhea

In Test Example 1, the stool property was monitored every day and rated on the 5-point scale of -: normal, ±: slightly soft stool, +: soft stool, ++: mild diarrhea, +++: diarrheal. The results are shown in Table 2.

**Table 2**

| | Animal No. | Day 0 (pretreatment) | Day 1 | Day 3 | Day 4 | Day 7 |
|---|---|---|---|---|---|---|
| Control | 1. | - | - | - | - | - |
| | 2. | - | - | - | - | - |
| | 3. | - | - | - | - | - |
| | 4. | - | - | - | - | - |
| | 5. | - | - | - | - | - |
| Compound B (116mg/kg) | 6. | - | +++ | +++ | + | - |
| | 7. | - | ++ | + + | ± | - |
| | 8. | - | +++ | +++ | ± | - |
| | 9. | - | + | +++ | + | - |
| Compound A (300mg/kg) | 10. | - | + | +++ | ± | ± |
| | 11. | - | - | ± | - | - |
| | 12. | - | ± | ± | - | - |
| | 13. | - | +++ | - | ± | - |
| -: normal stool, ±: slightly soft stool, +: soft stool, ++: mild diarrhea, +++: diarrhea | | | | | | |

It can be suggested from Table 2 that compound A, which is the compound of the invention, is less remarkable in diarrhea-inducing effect than compound B which is a known thrombolytic compound.

### BRIEF DESCRIPTION OF THE DRAWING

Fig. 1 shows the α2-PI decreasing activities of compound B and compound A. The abscissa represents time (hr) and the ordinate represents α2-PI activity (%) with the activity at 0 hr being taken as 100%. ○ represents data for control, ● data for compound B, and △ data for compound A.

## Claims

1. A pharmaceutical composition for antagonizing α₂-plasmin inhibitor which comprises a compound of the following general formula [I] or a physiologically acceptable salt thereof. 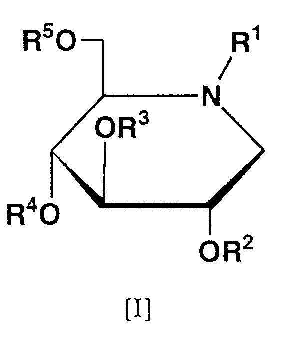 wherein R¹ represents hydrogen, alkyl, acyl, carboxyalkyl, alkyloxycarbonylalkyl, hydroxyalkyl, cycloalkylalkyl, arylalkyl, aryloxyalkyl, alkenyl, hydroxyalkenyl, arylalkenyl, aryloxyalkenyl, aryloxyarylalkenyl or alkylcarbamoylalkyl; R², R³, R⁴, and R⁵ may be the same or different and each represents hydrogen, alkyl, alkenyl or acyl (exclusive of the case in which all of R², R³, R⁴, and R⁵ represent hydrogen).

2. A pharmaceutical composition for promoting urokinase secretion which comprises the compound [I] or physiologically acceptable salt defined in Claim 1 as an active ingredient.

3. A pharmaceutical composition for the therapy or prophylaxis of thrombus which comprises the compound [I] or physiologically acceptable salt defined in Claim 1 as an active ingredient.

4. A pharmaceutical composition for antagonizing α₂-plasmin inhibitor which comprises a compound of the following general formula [II] or a physiologically acceptable salt thereof as an active ingredient. 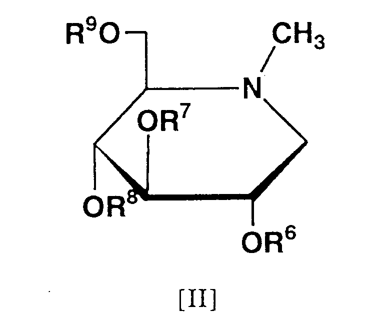 wherein R⁶, R⁷, R⁸, and R⁹ all represent the same C1-8 acyl group.

5. A pharmaceutical composition for promoting urokinase secretion which comprises the compound [II] or physiologically acceptable salt thereof defined in Claim 4 as an active ingredient.

6. A pharmaceutical composition for the therapy or prophylaxis of thrombus which comprises the compound [II] or physiologically acceptable salt thereof defined in Claim 4 as an active ingredient.
